Europäisches Patentamt

European Patent Office

Office européen des brevets

(19)

(11) EP 0 965 834 A1

## (12) DEMANDE DE BREVET EUROPEEN

(43) Date de publication:
**22.12.1999 Bulletin 1999/51**

(51) Int Cl.⁶: $G01N\ 19/02$
// $A61B5/103$

(21) Numéro de dépôt: **99401468.6**

(22) Date de dépôt: **15.06.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **18.06.1998 FR 9807711**

(71) Demandeurs:
- **LABORATOIRES DE BIOLOGIE VEGETALE YVES ROCHER**
  **56200 La Gacilly (FR)**
- **Asserin, Jérôme**
  **25030 Besançon (FR)**
- **Humbert, Philippe**
  **25030 Besançon (FR)**
- **Zahouani, Hassan**
  **69131 Ecully Cedex (FR)**

(72) Inventeurs:
- **Asserin, Jérôme,**
  **Sce. de Derma. Hôpital St-Jacques**
  **25030 Besançon (FR)**
- **Humbert, Philippe,**
  **Sce Derma. Hôpital St-Jacques**
  **25030 Besançon (FR)**
- **Zahouani, Hassan,**
  **Sce Derma. Hôpital St-Jacques**
  **25030 Besançon (FR)**
- **Couturaud, Virginie**
  **75008 Paris (FR)**
- **Mougin, Danielle**
  **92444 Issy Les Moulineaux Cedex (FR)**

(74) Mandataire:
**de la Bigne, Guillaume Michel Marie et al**
**c/o Cabinet Lavoix,**
**2, place d'Estienne d'Orves**
**75441 Paris Cedex 09 (FR)**

(54) **Dispositif d'évaluation des propriétés de friction d'une surface**

(57)     Ce dispositif comprend un palpeur (46), destiné à être appliqué au contact de la surface (S), porté par un ensemble (30) déformable sous l'effet de la friction entre le palpeur (46) et la surface (S). Le dispositif de mesure comprend de plus un support (22), formant fléau de balance, monté oscillant autour d'un axe d'appui (X) sensiblement horizontal. Ce support (22) comporte deux bras (22A,22B) s'étendant de part et d'autre de l'axe (X). Un premier bras (22A) porte l'ensemble déformable (30). Le second bras (22B) porte des moyens (32) d'équilibrage du support.

Application à l'évaluation in vivo de propriétés de friction de la peau, voire des ongles, des cheveux, des muqueuses ou des yeux, d'un être humain, ainsi que de tout support en reproduisant la surface.

FIG.2

EP 0 965 834 A1

## Description

La présente invention concerne un dispositif d'évaluation de propriétés de friction d'une surface.

Elle s'applique en particulier à l'évaluation in vivo de propriétés de friction de la peau d'un être humain afin de pouvoir quantifier les propriétés physiologiques et les effets de produits cosmétiques ou de médicaments.

On connaît déjà dans l'état de la technique un dispositif d'évaluation de propriétés de friction d'une surface, du type comprenant un palpeur, destiné à être appliqué au contact de la surface, porté par un ensemble déformable sous l'effet de la friction entre le palpeur et la surface.

Le mouvement relatif du palpeur et de la surface dont on veut évaluer des propriétés de friction est freiné par la friction entre cette surface et le palpeur. Ce dernier applique sur la surface une force de friction qui se décompose en une composante normale à la surface, encore appelée charge normale, et une composante tangente à la surface, encore appelée force tangentielle.

Lorsque le palpeur et la surface avec laquelle il est en contact sont en mouvement relatif, on définit habituellement le coefficient de friction dynamique $\mu_1$ de la surface par la relation suivante :

$$\mu_1 = \frac{F}{L}$$

dans laquelle F est le module de la force tangentielle et L est le module de la charge normale.

La force tangentielle qui précède immédiatement le mouvement relatif entre le palpeur et la surface est appelée habituellement force tangentielle limite. Le module $F_{lim}$ de cette force tangentielle limite, qui est généralement supérieur à celui de la force tangentielle lors du mouvement, permet de définir le coefficient de friction statique $\mu_2$ de la surface par la relation suivante.

$$\mu_2 = \frac{F_{lim}}{L}$$

La précision et la reproductibilité des mesures de $\mu_1$ et $\mu_2$ dépendent donc de la connaissance précise de la charge normale L appliquée par le palpeur sur la surface avec laquelle il est en contact. Or, dans le cas de la peau humaine, les dispositifs classiques d'évaluation des propriétés de friction, du type précité, ne permettent pas une maîtrise de la charge normale appliquée par le palpeur suffisante pour obtenir des mesures pertinentes et comparables des coefficients de friction de la peau.

L'invention a notamment pour but d'évaluer les coefficients de friction dynamique $\mu_1$ et statique $\mu_2$ de la peau d'un être humain vivant, ceci avec un dispositif du type précité permettant une très bonne maîtrise de la charge normale appliquée par le palpeur sur la peau.

A cet effet, l'invention a pour objet un dispositif d'évaluation de propriétés de friction d'une surface, du type précité, caractérisé en ce qu'il comprend un support, formant fléau de balance, monté oscillant autour d'un axe d'appui sensiblement horizontal, ce support comprenant deux bras, s'étendant de part et d'autre de l'axe, un premier bras portant l'ensemble déformable et le second bras portant des moyens d'équilibrage du support.

Suivant d'autres caractéristiques de ce dispositif :

- l'ensemble déformable comprend une embase sensiblement horizontale, portant le palpeur, reliée au support par deux lames souples sensiblement verticales et perpendiculaires à l'axe d'appui, de manière que l'embase et les lames délimitent sensiblement un prisme déformable ;
- les lames sont munies chacune d'une paire de jauges de contrainte, les deux paires de jauges de contrainte étant reliées électriquement entre elles par un montage en pont de Wheatstone ;
- le palpeur comprend une bille portée par une tige sensiblement verticale de liaison avec l'ensemble déformable;
- le premier bras du support porte des moyens de réception d'au moins une masselotte dont le poids correspond à une charge normale appliquée sur la surface par l'intermédiaire du palpeur ;
- la masselotte a une forme générale de disque percé en son milieu et les moyens de réception de la masselotte comprennent une broche, sensiblement verticale, coaxiale à la tige de liaison du palpeur, autour de laquelle est enfilée la masselotte ;
- l'axe d'appui est réglable en hauteur ;
- l'axe d'appui est matérialisé par un couteau solidaire du support en appui sur deux échancrures, formant paliers, ménagées respectivement dans deux traverses sensiblement horizontales ;
- la surface est portée par une platine mobile en translation sensiblement horizontalement et parallèlement à l'axe d'appui.

L'invention a également pour objet l'application du dispositif précité à l'évaluation de propriétés de friction de la surface de la peau d'un être humain vivant et plus particulièrement à l'évaluation de propriétés de friction de la surface de la peau recouvrant la partie interne de l'avant-bras d'un être humain vivant.

Suivant encore d'autres caractéristiques du dispositif selon l'invention considéré dans son application précédente :

- le dispositif comprend un boîtier de logement et d'immobilisation de l'avant-bras, porté par la platine, muni d'une ouverture d'accès du palpeur à une partie sensiblement plane de la surface de la peau ;

- le boîtier comprend une gouttière de positionnement de l'avant-bras, une poignée sur laquelle peut s'agripper la main prolongeant l'avant-bras et un sac gonflable de plaquage de l'avant-bras contre les bords de l'ouverture d'accès.

L'invention sera mieux comprise à la lecture de la description qui va suivre donnée uniquement à titre d'exemple et faite en se référant aux dessins dans lesquels:

- la figure 1 est une vue en perspective d'un dispositif d'évaluation de propriétés de friction d'une surface, selon l'invention ;
- la figure 2 est une vue de face d'un module de mesure du dispositif représenté à la figure 1 ;
- la figure 3 est une vue de dessus du module représenté à la figure 2 ;
- la figure 4 est une vue de côté, suivant la flèche 4 de la figure 2 ;
- la figure 5 est une vue suivant la flèche 5 de la figure 1 du boîtier d'immobilisation d'un avant-bras d'être humain comportant un couvercle représenté en position entrouverte ;
- les figures 6 et 7 sont des vues de dessus du boîtier représenté à la figure 5, le couvercle de ce dernier n'étant pas représenté à la figure 6 et étant représenté en position fermée à la figure 7.

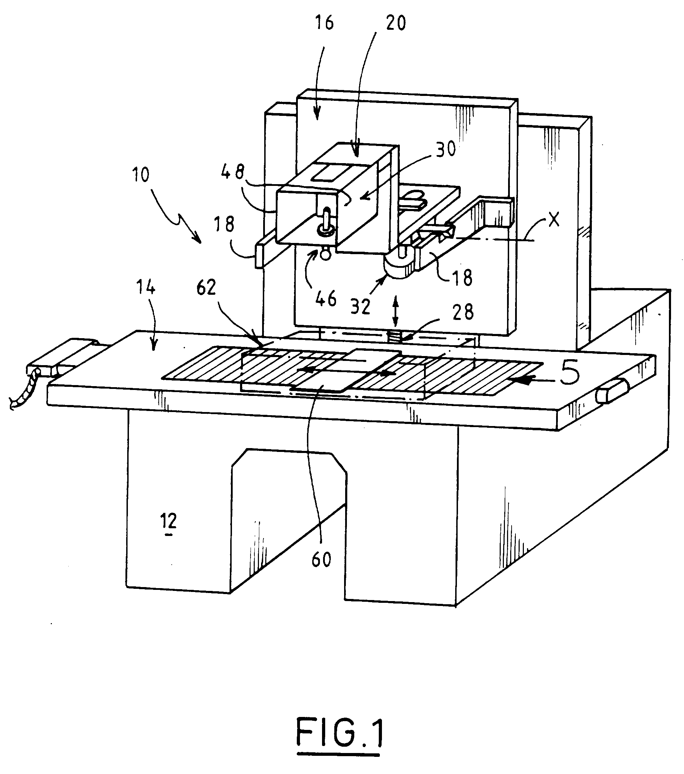

On a représenté sur la figure 1 un dispositif d'évaluation de propriétés de friction d'une surface, selon l'invention, désigné par la référence générale 10.

Ce dispositif 10 est destiné plus particulièrement à évaluer in vivo des propriétés de friction de la peau humaine, notamment à mesurer les coefficients de friction dynamique $\mu_1$ et statique $\mu_2$ définis précédemment.

Le dispositif 10 comprend un bâti 12 portant une table de déplacement motorisée 14 sensiblement horizontale et un châssis 16, formant potence. Le châssis 16 comporte deux traverses 18, sensiblement horizontales et parallèles entre elles, portant un module de mesure oscillant 20.

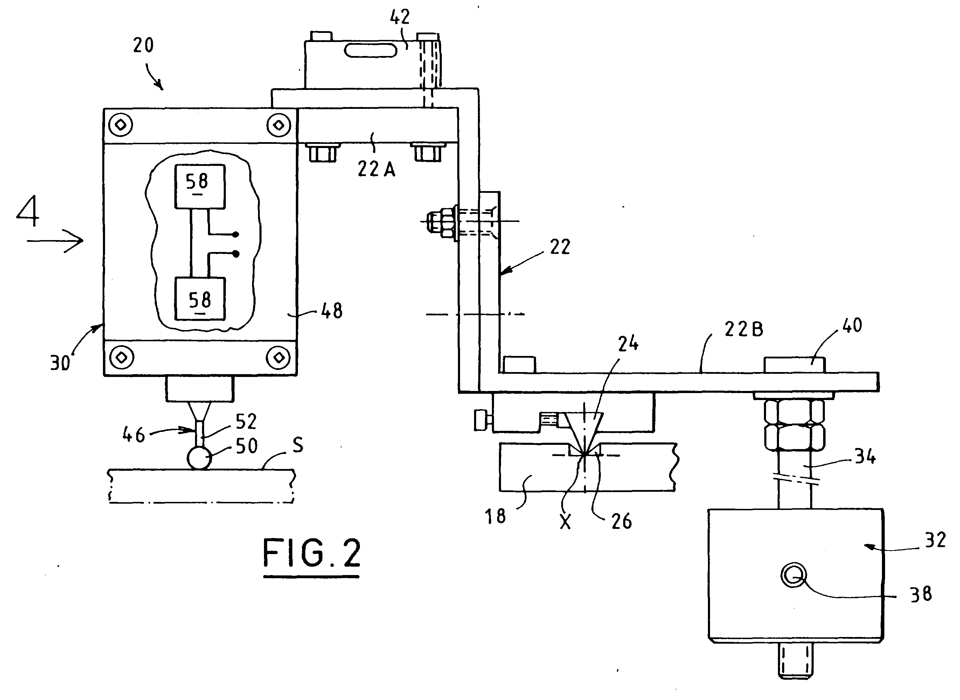

Ce module 20, qui est représenté plus en détail sur les figures 2 à 4, comporte un support 22 doublement coudé, formant fléau de balance, monté oscillant autour d'un axe d'appui X sensiblement horizontal et perpendiculaire aux traverses 18. Cet axe d'appui X est matérialisé par un couteau 24, solidaire du support 22, en appui sur deux échancrures 26, formant paliers, ménagées respectivement dans les deux traverses 18.

Le châssis 16 est relié au bâti 12 par l'intermédiaire de moyens classiques 28 de réglage en hauteur (voir figure 1) permettant le réglage en hauteur de l'axe X. Ces moyens 28 sont éventuellement motorisés.

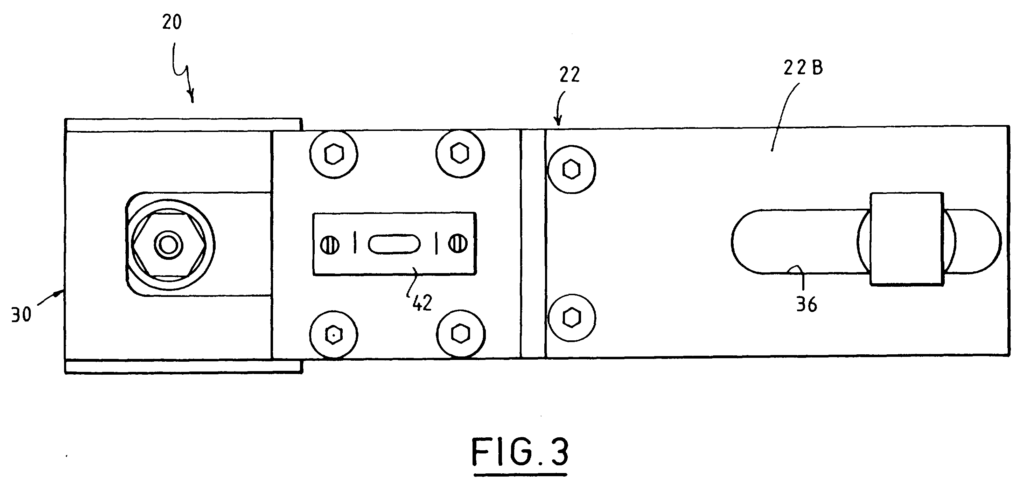

Le support 22 comporte deux bras 22A,22B s'étendant de part et d'autre de l'axe X. Un premier bras 22A porte un ensemble déformable 30. Le second bras 22B porte des moyens d'équilibrage du support comprenant, de préférence, un contrepoids 32 monté déplaçable axialement, par vissage ou coulissement, sur une tige 34 sensiblement verticale reliée à ce second bras 22B. De préférence, la tige 34 est montée coulissante, sensiblement horizontalement et transversalement à l'axe X dans une boutonnière 36 ménagée dans le second bras 22B (voir figure 3).

Des moyens classiques 38,40 permettent d'immobiliser le contrepoids 32 par rapport à la tige 34 et cette tige 34 par rapport à la boutonnière 36 dans des positions de réglage souhaitées.

Un niveau à bulle 42, fixé sur le premier bras 22A, permet de vérifier l'horizontalité du support 22.

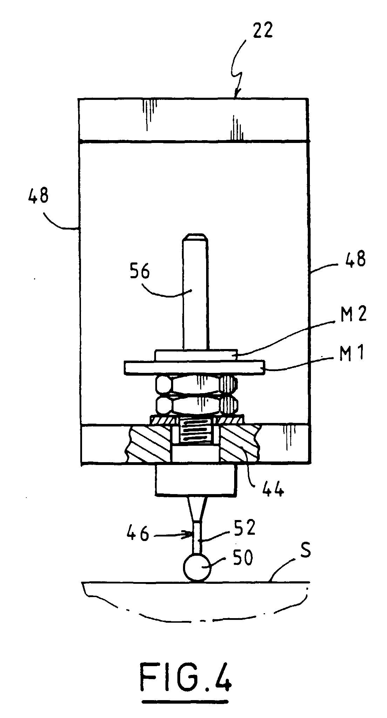

L'ensemble déformable 30 comprend une embase rigide 44 sensiblement horizontale (voir notamment figure 4), portant un palpeur 46. L'embase 44 est reliée au premier bras 22A du support par deux lames souples 48, sensiblement verticales et perpendiculaires à l'axe d'appui X. L'embase 44 et les lames 48 délimitent ainsi sensiblement un prisme qui est déformable sous l'effet de la friction entre le palpeur 46 et une surface S avec laquelle il est en contact.

De préférence, le capteur 46 comporte une bille 50 de contact avec la surface S portée par une tige 52 sensiblement verticale de liaison avec la face inférieure de l'embase 44.

Le diamètre de la bille 50 est par exemple égal à 1,5 ou 3 mm.

Une broche 56 sensiblement verticale (voir figure 4), reliée à la face supérieure de l'embase 44 de façon coaxiale à la tige de liaison 52, est destinée à recevoir des disques percés en leur milieu formant des masselottes M1,M2. Le poids de ces masselottes correspond pratiquement à la charge normale appliquée par le palpeur 46 sur la surface S. Cette charge normale est donc réglée en enfilant et empilant le nombre requis de masselotte autour de la broche 56.

Les lames 48, par exemple en bronze de béryllium, sont munies chacune d'une paire de jauges de contrainte 58. Les quatre jauges 58 sont reliées électriquement entre elles par un montage en pont de Wheatstone.

La déformation des lames 48, mesurée par les jauges de contrainte 58, permet de déduire la force tangentielle F ou $F_{lim}$ exercée par le palpeur 46 sur la surface S.

La table 14, de type classique, est munie d'une platine 60 mobile en translation, sensiblement horizontalement et parallèlement à l'axe X. De préférence, la platine 60 est du type à déplacements micrométriques.

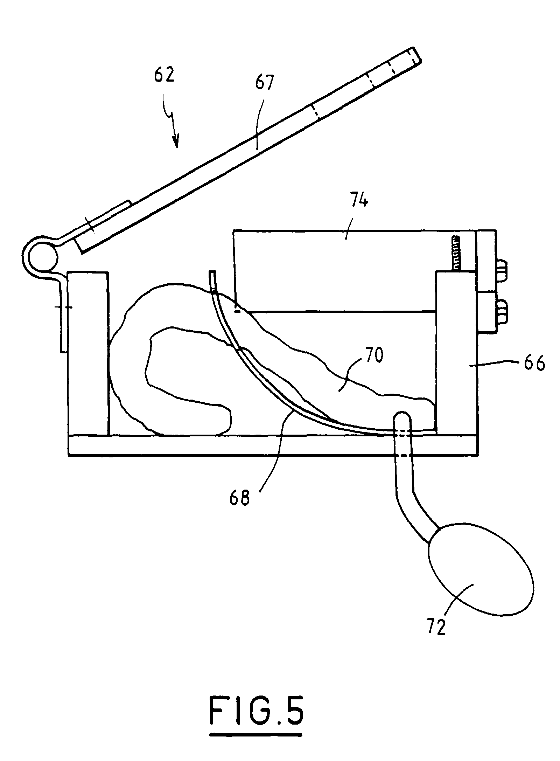

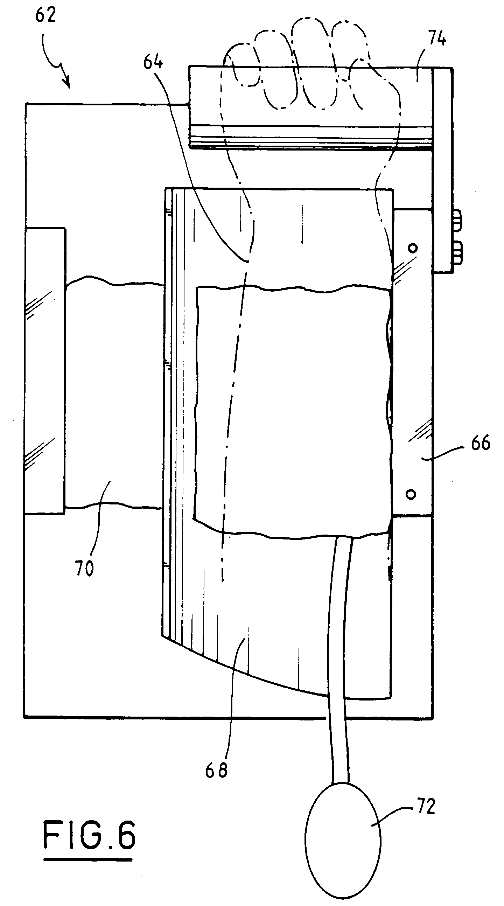

Un boîtier 62, représenté plus en détail sur les figures 5 à 7, est accroché de façon connue en soi sur la platine 60.

Le boîtier 62 est destiné à loger et immobiliser l'avant-bras 64 d'un être humain afin de permettre l'évaluation in vivo de propriétés de friction de la peau recouvrant la partie interne de cet avant-bras.

Le boîtier 62 comporte un corps 66 de logement de l'avant-bras 64 partiellement obturable par un couvercle 67.

Une gouttière 68 de positionnement de l'avant-bras 64 est logée dans le corps 66. Un sac gonflable 70 est également logé dans le corps 66 de manière à s'étendre à travers la gouttière 68, au dessus et en dessous de celle-ci.

Le sac 70 est gonflé de façon connue en soi, à l'aide d'une poire 72. Le fonctionnement du sac 70 est tout à fait similaire à celui équipant un tensiomètre médical classique.

Pour faciliter le positionnement de l'avant-bras 64 dans la gouttière 68 et le corps 66, ce dernier est muni d'une poignée 74 sur laquelle peut s'agripper la main prolongeant l'avant-bras 64.

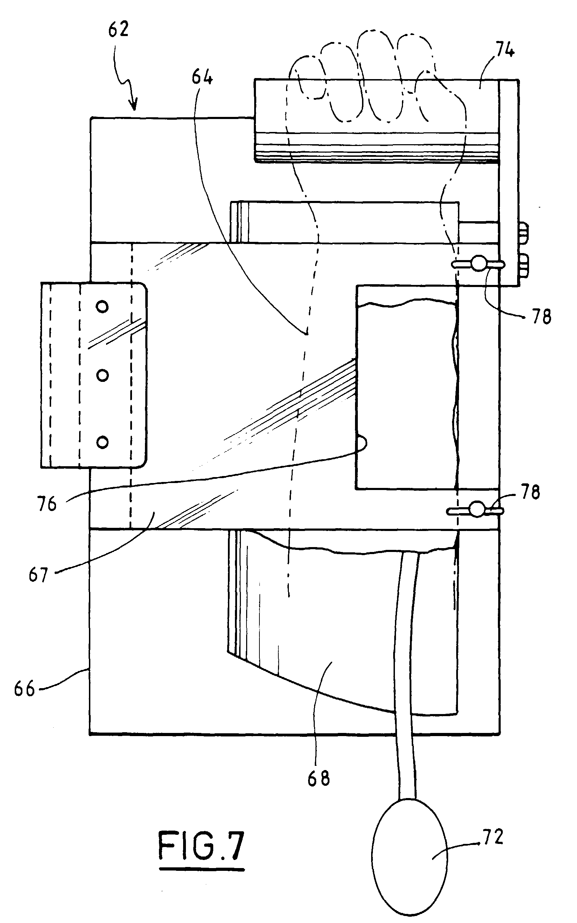

Le couvercle 67 est muni d'une ouverture 76 (voir figure 7) d'accès du palpeur 46 à une partie sensiblement plane de la surface S de la peau de la partie interne du bras.

Le couvercle 67 est maintenu en position fermée par exemple à l'aide de moyens de vissage 78 à papillon.

Le dispositif 10 selon l'invention est simple à utiliser.

Initialement, la personne sur laquelle on veut évaluer des propriétés de friction de la peau place son avant-bras dans le corps 66 du boîtier en saisissant la poignée 74, de manière à présenter la partie interne de l'avant-bras vers le haut. Puis on referme le couvercle 67. La partie interne de l'avant-bras est plaquée contre les bords de l'ouverture 76 par gonflage du sac 70. On notera que lors de ce gonflage, la gouttière 68 est enserrée entre les deux parties du sac 70 s'étendant au dessus et en dessous d'elle. Du fait du plaquage de l'avant-bras 64 contre l'ouverture 76, la surface de la peau visible à travers cette ouverture 76 est sensiblement horizontale.

Par ailleurs, on règle la hauteur de l'axe X, à l'aide des moyens 28, de manière à placer le palpeur 46 au niveau de l'ouverture 76, et on équilibre le support 22 en réglant horizontalement et verticalement la position du contrepoids 32. L'horizontalité du support 22 est vérifiée grâce au niveau à bulle 42.

Enfin, le palpeur 46 étant en contact avec la peau à travers l'ouverture 76, on empile le nombre souhaité de masselottes M1,M2 sur la broche 56 pour imposer une charge normale prédéterminée sur la surface S de la peau.

Pour déterminer les coefficients de friction $\mu_1$ et $\mu_2$, on commande le déplacement de la platine 60 afin d'entraîner le boîtier 62 en translation. Sous l'effet de la force tangentielle exercée par le palpeur 46 sur la peau avec laquelle il est en contact, les lames 48 se déforment par pliage dans le sens de déplacement du boîtier 62.

Le signal électrique aux bornes du pont de Wheatstone, quantifiant la déformation des lames, est traité de manière à déduire le module F ou $F_{lim}$ de la force tangentielle et, connaissant le module L de la charge normale (poids des masselottes M1,M2), les coefficients de friction $\mu_1$ et $\mu_2$ d'après les relations précisées précédemment.

Le pilotage de la platine 60 et le traitement des données provenant notamment du pont de Wheatstone sont effectués à l'aide de moyens classiques notamment informatiques.

L'invention ne se limite pas au mode de réalisation décrit ci-dessus. En particulier, elle peut s'appliquer à la mesure de coefficients de friction de surfaces très variées autres que la surface de la peau humaine, notamment la surface des ongles, des cheveux, des muqueuses ou des yeux d'un être humain ou encore la surface de ces éléments reproduite sur un support quelconque.

Parmi les avantages de l'invention, on notera les suivants.

Le dispositif selon l'invention permet de connaître de façon très précise, au millième de Newton près, la charge normale appliquée par le palpeur sur la surface dont on veut déterminer des propriétés de friction.

Le contact linéaire du couteau 24 sur les traverses 18 se fait quasiment sans frottement, si bien que le poids des masselottes empilées sur la broche 56 de l'ensemble déformable 30 est transmis, via le palpeur, à la surface S, sous la forme d'une charge normale de valeur pratiquement identique au poids de cette masselotte.

La connaissance très précise de la charge normale appliquée à la surface permet de déduire de façon très fiable et précise le coefficient de frottement de la surface.

**Revendications**

1. Dispositif d'évaluation de propriétés de friction d'une surface (S), du type comprenant un palpeur (46), destiné à être appliqué au contact de la surface (S), porté par un ensemble (30) déformable sous l'effet de la friction entre le palpeur (46) et la surface (S), **caractérisé en ce qu'**il comprend un support (22), formant fléau de balance, monté oscillant autour d'un axe d'appui (X) sensiblement horizontal, ce support (22) comprenant deux bras (22A, 22B), s'étendant de part et d'autre de l'axe (X), un premier bras (22A) portant l'ensemble déformable (30) et le second bras (22B) portant des moyens (32) d'équilibrage du support (22).

2. Dispositif selon la revendication 1, caractérisé en ce que l'ensemble déformable (30) comprend une embase (44) sensiblement horizontale, portant le palpeur (46), reliée au support (22) par deux lames souples (48) sensiblement verticales et perpendiculaires à l'axe d'appui (X), de manière que l'embase (44) et les lames (48) délimitent sensiblement un prisme déformable.

3. Dispositif selon la revendication 2, caractérisé en ce que les lames (48) sont munies chacune d'une paire de jauges de contrainte (58), les deux paires de jauges de contrainte étant reliées électriquement entre elles par un montage en pont de Wheatstone.

4. Dispositif selon l'une quelconque des revendications précédentes, caractérisé en ce que le palpeur (46) comprend une bille (50) portée par une tige (52) sensiblement verticale de liaison avec l'ensemble déformable (30).

5. Dispositif selon l'une quelconque des revendications précédentes, caractérisé en ce que le premier bras (22A) du support porte des moyens (56) de réception d'au moins une masselotte (M1,M2) dont le poids correspond à une charge normale appliquée sur la surface (S) par l'intermédiaire du palpeur (46).

6. Dispositif selon les revendications 2,4 et 5, les trois prises ensemble, caractérisé en ce que la masselotte (M1,M2) a une forme générale de disque percé en son milieu et en ce que les moyens de réception de la masselotte (M1,M2) comprennent une broche (56), sensiblement verticale, coaxiale à la tige de liaison (52) du palpeur, autour de laquelle est enfilée la masselotte (M1,M2).

7. Dispositif selon l'une quelconque des revendications précédentes, caractérisé en ce que l'axe d'appui (X) est réglable en hauteur.

8. Dispositif selon l'une quelconque des revendications précédentes, caractérisé en ce que l'axe d'appui (X) est matérialisé par un couteau (24) solidaire du support (22) en appui sur deux échancrures (26), formant paliers, ménagées respectivement dans deux traverses sensiblement horizontales.

9. Dispositif selon l'une quelconque des revendications précédentes, caractérisé en ce que la surface (S) est portée par une platine (60) mobile en translation sensiblement horizontalement et parallèlement à l'axe d'appui (X).

10. Application d'un dispositif selon l'une quelconque des revendications 1 à 9 à l'évaluation de propriétés de friction de la surface de la peau d'un être humain vivant.

11. Application du dispositif selon l'une quelconque des revendications 1 à 9 à l'évaluation de propriétés de friction de la surface de la peau recouvrant la partie interne de l'avant-bras (64) d'un être humain vivant.

12. Dispositif selon la revendication 9, considéré dans son application selon la revendication 11, caractérisé en ce qu'il comprend un boîtier (62) de logement et d'immobilisation de l'avant-bras (64), porté par la platine (60), muni d'une ouverture (76) d'accès du palpeur (46) à une partie sensiblement plane de la surface de la peau.

13. Dispositif selon la revendication 12, caractérisé en ce que le boîtier (62) comprend une gouttière (68) de positionnement de l'avant-bras (64), une poignée (74) sur laquelle peut s'agripper la main prolongeant l'avant-bras (64) et un sac gonflable (70) de plaquage de l'avant-bras (64) contre les bords de l'ouverture d'accès (76).

# FIG.1

FIG.2

FIG.3

EP 0 965 834 A1

# FIG.4

FIG.5

# FIG.6

FIG.7

Office européen
des brevets

**RAPPORT DE RECHERCHE EUROPEENNE**

Numéro de la demande

EP 99 40 1468

| | **DOCUMENTS CONSIDERES COMME PERTINENTS** | | |
|---|---|---|---|
| Catégorie | Citation du document avec indication, en cas de besoin, des parties pertinentes | Revendication concernée | CLASSEMENT DE LA DEMANDE (Int.Cl.6) |
| A | ALLISTON-GREINER A F: "Friction test machines for rubbery materials" TRIBOTEST SEP 1994 LEAF COPPIN PUBL LTD, DEAL, ENGL, vol. 1, no. 1, septembre 1994 (1994-09), pages 63-75, XP002094975 * page 64 - page 66; figures 1,2 * --- | 1,4-7, 9-11 | G01N19/02 //A61B5/103 |
| A | US 3 033 019 A (M A OLIVER) 8 mai 1962 (1962-05-08) * colonne 2, ligne 39 - colonne 3, ligne 36; figure 1 * --- | 1-4 | |
| A | DE 295 14 273 U (SCHNEIDER GOTTFRIED PROF DR IN) 29 février 1996 (1996-02-29) ----- | | |
| | | | **DOMAINES TECHNIQUES RECHERCHES (Int.Cl.6)** |
| | | | G01N A61B |
| | Le présent rapport a été établi pour toutes les revendications | | |

| Lieu de la recherche | Date d'achèvement de la recherche | Examinateur |
|---|---|---|
| LA HAYE | 23 septembre 1999 | Hodson, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**     EP 99 40 1468

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-09-1999

| Document brevet cité au rapport de recherche | Date de publication | Membre(s) de la famille de brevet(s) | Date de publication |
|---|---|---|---|
| US 3033019 A | 08-05-1962 | AUCUN | |
| DE 29514273 U | 29-02-1996 | AUCUN | |

EPO FORM P0460

Pour tout renseignement concernant cette annexe : voir Journal Officiel de l'Office européen des brevets, No.12/82